# EUROPEAN PATENT SPECIFICATION

(11) **EP 1 158 989 B1**
(45) Date of publication and mention of the grant of the patent: **20.06.2007**
(21) Application number: 00916915.2
(22) Date of filing: 09.03.2000
(51) Int. Cl.: A61K 31/575, A61P 9/04

(54) **USE OF INHIBITORS OF ENDOTOXIN FOR THE TREATMENT OF CACHEXIA**
ENDOTOXINHEMMERN ZUR BEHANDLUNG VON KACHEXIE
UTILISATION D'INHIBITEURS DES ENDOTOXINES POUR LE TRAITEMENT DE LA CACHEXIE

(30) Priority: 09.03.1999 GB 9905315; 09.03.1999 GB 9905300; 09.03.1999 GB 9905310; 09.03.1999 GB 9905307; 09.03.1999 GB 9905314
(43) Date of publication of application: 05.12.2001
(73) Proprietor: Anker, Stefan, 13053 Berlin (DE); Volk, Hans-Dieter, 10178 Berlin (DE); Schumann, Ralf Reiner, 13407 Berlin (DE); Coats, Andrew, Woollahra NSW 2025 (AU)
(72) Inventor: VOLK, Hans-Dieter, D-10178 Berlin (DE); PLAUTH, Mathias, D-10709 Berlin (DE); LOCHS, Herbert, D-10717 Berlin (DE)
(74) Representative: Baumbach, Friedrich
(86) International application number: PCT/EP2000/002062
(87) International publication number: WO 2000/053165

(56) References cited:
- SASATOMI K ET AL: "Effects of ursodeoxycholic acid on intrahepatic localization of endotoxin in bile duct-ligated rats and patients with primary biliary cirrhosis." GASTROENTEROLOGY, vol. 110, no. 4 SUPPL., 1996, page A1312 XP000971586 96th Annual Meeting of the American Gastroenterological Association and the Digestive Disease Week;San Francisco, California, USA; May 19-22, 1996 ISSN: 0016-5085
- SASATOMI KURUMI ET AL: "Abnormal accumulation of endotoxin in biliary epithelial cells in primary biliary cirrhosis and primary sclerosing cholangitis." JOURNAL OF HEPATOLOGY, vol. 29, no. 3, 1998, pages 409-416, XP000971565 ISSN: 0168-8278
- SASATOMI K ET AL: "Effects of ursodeoxycholic acid on intrahepatic localization of endotoxin in patients with primary biliary cirrhosis." JOURNAL OF ENDOTOXIN RESEARCH, vol. 3, no. SUPPL. 1, 1996, page 25 XP000971569 Fourth International Endotoxin Society Conference;Nagoya, Japan; October 22-25, 1996 ISSN: 0968-0519
- POO JORGE L ET AL: "Effects of ursodeoxycholic acid on hemodynamic and renal function abnormalities induced by obstructive jaundice in rats." RENAL FAILURE, vol. 17, no. 1, 1995, pages 13-20, XP000971578 ISSN: 0886-022X
- GREVE J W ET AL: "BILE ACIDS INHIBIT ENDOTOXIN-INDUCED RELEASE OF TUMOR NECROSIS FACTOR BY MONOCYTES AN IN-VITRO STUDY" HEPATOLOGY, vol. 10, no. 4, 1989, pages 454-458, XP000971557 ISSN: 0270-9139
- ANKER STEFAN D ET AL: "Elevated soluble CD14 receptors and altered cytokines in chronic heart failure." AMERICAN JOURNAL OF CARDIOLOGY, vol. 79, no. 10, 1997, pages 1426-1430, XP000971579 ISSN: 0002-9149
- ANKER STEFAN D ET AL: "Soluble CD14 receptors and cytokines in chronic heart failure: Immune activation due to endotoxin?" EUROPEAN HEART JOURNAL, vol. 18, no. ABSTR. SUPPL., 1997, page 297 XP000971584 XIXth Congress of the European Society of Cardiology together with the 32nd Annual General Meeting of the Association of European Paediatric Cardiologists;Stockholm, Sweden; August 24-28, 1997 ISSN: 0195-668X
- EGERER KARL R ET AL: "Soluble CD14 receptors are increased in cachectic chronic heart failure suggesting a role for endotoxin." CIRCULATION, vol. 94, no. 8 SUPPL., 1996, pages I672-I673, XP000971568 69th Scientific Sessions of the American Heart Association;New Orleans, Louisiana, USA; November 10-13, 1996 ISSN: 0009-7322
- SAKISAKA S ET AL: "Biliary secretion of endotoxin and pathogenesis of primary biliary cirrhosis." YALE JOURNAL OF BIOLOGY AND MEDICINE, vol. 70, no. 4, July 1997 (1997-07), pages 403-408, XP000971577 ISSN: 0044-0086

## Description

The present invention relates to therapy and the use of agents in the therapy of cachexia and wasting syndromes due to diseases other than congestive heart failure. Cachexia occurs in a number of other chronic diseases, like liver cirrhosis, chronic obstructive pulmonary disease, chronic renal failure, diabetes, rheumatoid arthritis. Cachexia and weight loss are linked to inflammatory processes and they are linked to increased mortality and/or morbidity. Cytokine activation is a potential causal mechanism for the development of cachexia also in these other diseases.

No one has previously proposed that one or all of the following agents may be useful in the management of patients with cachexia due to liver cirrhosis, chronic obstructive pulmonary disease, chronic renal failure, diabetes, rheumatoid arthritis:
- a bile acid,
- BPI,
- LPS binding protein
- an antibody capable of binding to endotoxin,
- lipoproteins
- an antibody able to bind the CD14 receptor,
- a soluble CD 14 receptor.

The following classes of patients in particular may benefit from treatment

Patients with body wasting or cachexia due to liver cirrhosis, chronic obstructive pulmonary disease, chronic renal failure, diabetes, rheumatoid arthritis.

It is preferred that the patient has cachexia, as characterised by loss of muscle, fat, and or bone tissue.

It is preferred that the patient has experienced weight loss >7.5%.

It is preferred that the compound is able to substantially reduce the biological activity of endotoxin (lipopolysaccharide) such that the endotoxin mediated production of inflammatory cytokines in the circulating blood is reduced.

By "bile acid" we include all naturally occurring bile acids whether from man or from another animal. Also is included bile acids which are synthetic or semi-synthetic derivatives of naturally occurring bile acids. Of course, all bile acids including those that are "naturally occurring" may be synthesised chemically.

Bile acids are available from Falk Pharma GmbH and are described, for example, in WP96/17859, DE29717252 and WO98/05339.

Bile acids for use in the method of the invention include, but are not limited to, chemodeoxycholic acid (3α, 7α - dihydroxy-5-cholan-24-oic acid), arsodeoxycholic acid (3α, 7-dihydroxy-5-cholan-24-oic acid), dehydrocholic acid (3,7,12-trioxo-5-cholan-24-oic acid), cholic acid and deoxycholic acid.

Preferably, the bile acid is a bile acid which is able to form micelles. Preferably, the bile acid is able to form a micelle around an endotoxin (lipopolysacharide molecule). It is particularly preferred that the bile acid is able to bind to endotoxin (lipopolysaccharide) molecules and substantially reduce the available endotoxin in the patient. In particular, it is preferred if the bile acid is able to substantially reduce the biological activity of endotoxin (lipopolysaccharide) such that the endotoxin has a substantially reduced effect on the liver or does not reach the liver in a substantially active form.

It is preferred if the bile acid is any one of ursodeoxycholic acid, chemodeoxycholic acid, dehydrocholic acid, cholic acid and deoxycholic acid.

It is preferred if the bile acid is ursodeoxycholic acid.

Originally, UDCA was registered for the medical treatment of gallstones (Leuschner et al. Our ten year experience in gallstone dissolution. Comparison with the national Canadian gallstone (NCGS, USA) and the Toky co-operative gallstone study (TCGS, Japan). Gastroenterology 1982, 82:1113). Ursodeoxycholic acid has for many years been proposed to be useful also in patients with cholestatic disease, and particularly in patients with primary biliary cirrhosis, a chronic cholestatic liver disease (Lindor et al. Effects of ursodeoxycholic acid on survival in patients with primary biliary cirrhosis. Gastroenterology 1996, 110:1515-1518). In analogy, UDCA is used in other cholestatic disorders like primary sclerosing cholangitis (Beuerset al: Therapie der autoimmunen Hepatitis, primär biliären Zirrhose und primär sklerosierenden Cholangitis. Konsensus der Deutschen Gesellschaft für Verdauungs- und Stoffwechselkrankheiten. Z. Gastroenterologie 1997; 35:1041-1049) or benign cholestasis of pregnancy (Palma et al. Ursodeoxycholic acid in the treatment of cholestasis of pregnancy: a randomized, double-blind study controlled with placebo. J Hepatol 1997, 27:1022-1028). Regarding its mode of action, most authorities regard increased bile flow and a reduced hepatocellular insult as a result of improved bile flow and altered bile salt patterns as the main modes of UDCA action in chronic cholestatic liver diseases.

However, a very recent meta-analysis concluded that "Published randomised controlled trials of UDCA do not show evidence of therapeutic, benefit in primary biliary cirrhosis and its use as standard therapy needs to be re-examined." (Goulis et al. Randomised controlled trials of ursodeoxycholic-acid therapy for primary biliary cirrhosis: a meta-analysis. Lancet 1999 Sep 25;354:1053-1060.)

As for other liver diseases another recent review article concluded "Ursodeoxycholic acid is of unproven efficacy in non-cholestatic disorders such as acute rejection after liver transplantation, non-alcoholic steatohepatitis, alcoholic liver disease and chronic viral hepatitis." Trauner M and Graziadei IW. Review article: mechanisms of action and therapeutic applications of ursodeoxycholic acid in chronic liver diseases. Aliment Pharmacol Ther. 1999 Aug; 13(8): 979-996.

Therefore, treatment with ursodeoxycholic acid (UDCA) can not be considered a treatment with proven efficacy in patients with liver disease.

It has never been suggested that ursodeoxycholic acid (UDCA) should be specifically given to patients with cachexia due to liver cirrhosis.

It has never been suggested that ursodeoxycholic acid (UDCA) should be specifically given to patients with alcoholic liver cirrhosis. In fact, such patients were specifically excluded from studies.

Alterations in nutritional state leading to abnormal body composition are detectable already in early stages of liver cirrhosis and are clinically overt in the great majority of patients with advanced disease. Despite the well accepted prognostic role of cachexia or protein-energy-malnutrition in cirrhosis its pathogenesis is not fully understood. Although alcohol abuse and inadequate nutrient composition may play some role in patients with alcoholic liver disease this clearly is not operative in patients with liver disease of other etiology in whom malnutrition is as great a problem as in those with alcoholic liver disease (Plauth et al: ESPEN guidelines for nutrition in liver disease and transplantation. Clin Nutr 1997, 16:43-55). Nutrient intake is reduced in many patients with advanced liver cirrhosis and does not match requirements. It is unknown, however, whether food intake is reduced as a consequence of mechanical factors such as ascites or due to altered appetite regulation or other processes.

It is long known that endotoxaemia occurs in a number of patients with liver cirrhosis. It is not known, whether endotoxin (LPS) levels are particularly raised in patients with cachexia due to liver cirrhosis.

Depending of the severity of the liver cirrhosis process, cachexia occurs in 30 to 60% of patients with liver cirrhosis, and the survival of patients with cachexia in liver cirrhosis is impaired. (Plauth et al: ESPEN guidelines for nutrition in liver disease and transplantation. Clin Nutr 1997, 16:43-55). There is no known specific therapy for these patients, and randomised placebo controlled clinical trials to reverse the cachexia in liver cirrhosis patients, and particularly in those with alcohol induced liver cirrhosis have not been performed. Additionally, patients with a body cell mass (BCM) < 35% of body weight have reduced survival also after liver transplantation, and the 5-year survival rate is 54% compared to 88% in patients with BCM >35% (p < .01) (Selberg et al. Identification of high- and low-risk patients before liver transplantation: a prospective cohort study of nutritional and metabolic parameters in 150 patients. Hepatology 1997;25:652-657).

It has also been suggested that bile acids can protect the liver against endotoxin action in obstructive jaundice when patients undergo surgery (Greve et al. Bile acids inhibit endotoxin-induced release of tumor necrosis factor by monocytes: an in vitro study. Hepatology 1989 Oct;10(4):454-458). With regards to monocyte generated cytokine production in response to LPS, in this study deoxycholic acid was the most effective, chenodeoxycholic acid was less effective and ursodeoxycholic acid was ineffective in the concentrations used. Bile acids did not inactivate endotoxin as measured in a chromogenic Limulus amebocyte lysate assay. In these studies patients with non-cholestatic or alcoholic aetiology were not considered, and there was no data or discussion of cachexia and weight loss.

In experiments, rats with obstructive jaundice, LPS was administered via the portal vein. In UDCA-treated rats, the endotoxin concentration was significantly lower, however, that UDCA had no effect on the TNF-alpha levels (Hori Y & Ohyanagi H. Protective effect of the intravenous administration of ursodeoxycholic acid against endotoxaemia in rats with obstructive jaundice. Surg-Today 1997;27:140-144). In a case control study UDCA showed also no clinical benefit in patients with chronic hepatitis C, and serum TNF and IL-6 levels could not be shown to be affected by UDCA treatment (Lu et al. Efficacy of ursodeoxycholic acid in the treatment of patients with chronic hepatitis C. J Gastroenterol Hepatol 1995;10:432-437.

In summary, the immunological effects of ursodeoxycholic acid (UDCA) on plasma LPS and cytokine levels are poor in these studies, and the cellular effects of ursodeoxycholic acid (UDCA) are conflicting.

It is important to note that it has never been proposed that ursodeoxycholic acid (UDCA) should be given in patients with weight loss, i.e. cachexia, in patients with liver disease. It has never been proposed that ursodeoxycholic acid (UDCA) could prevent or reverse weight loss, i.e. cachexia, in patients with liver disease. Additionally, it has never been proposed that ursodeoxycholic acid (UDCA) could prevent or reverse weight loss, i.e. cachexia, in patients with chronic obstructive pulmonary disease, chronic renal failure, diabetes, rheumatoid arthritis.

The invention will now be described by reference to the following additional examples and figures.

### Example 1:

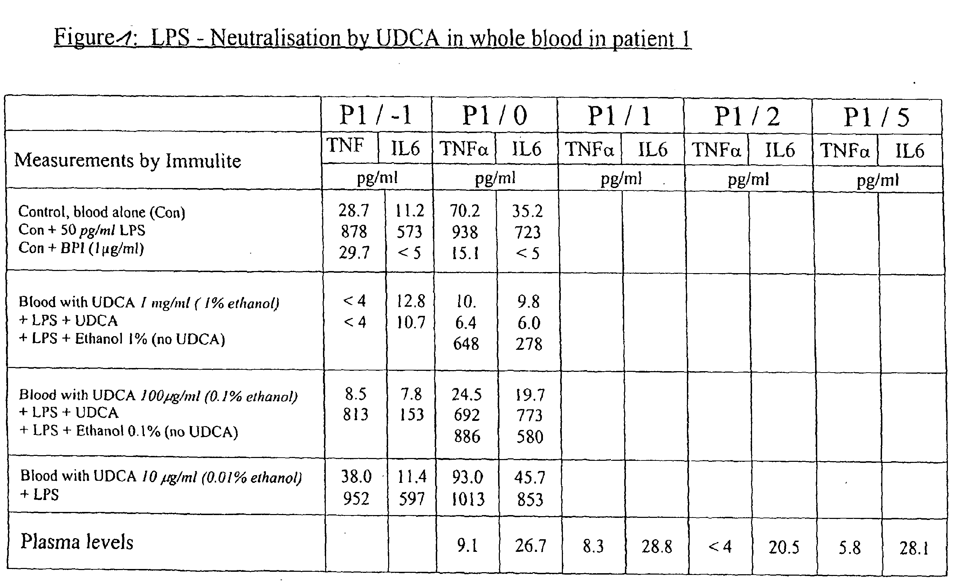

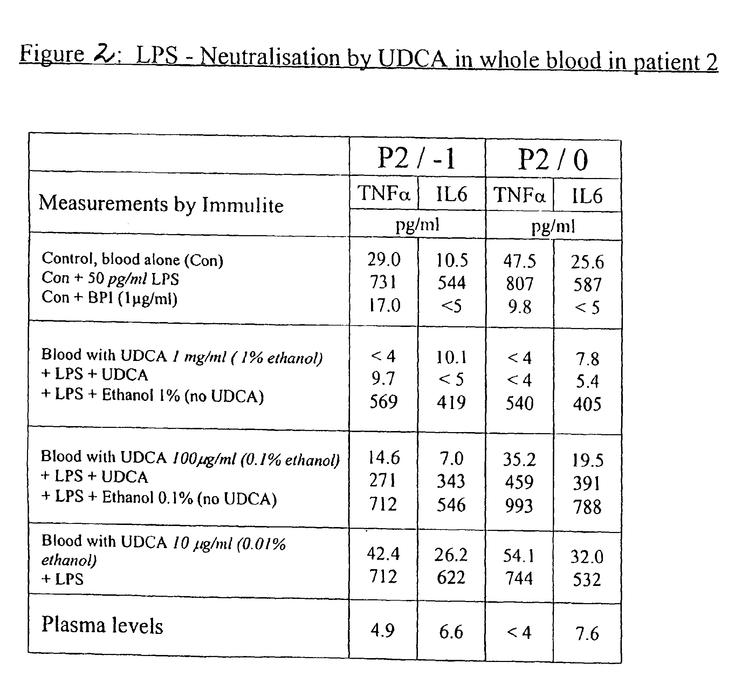

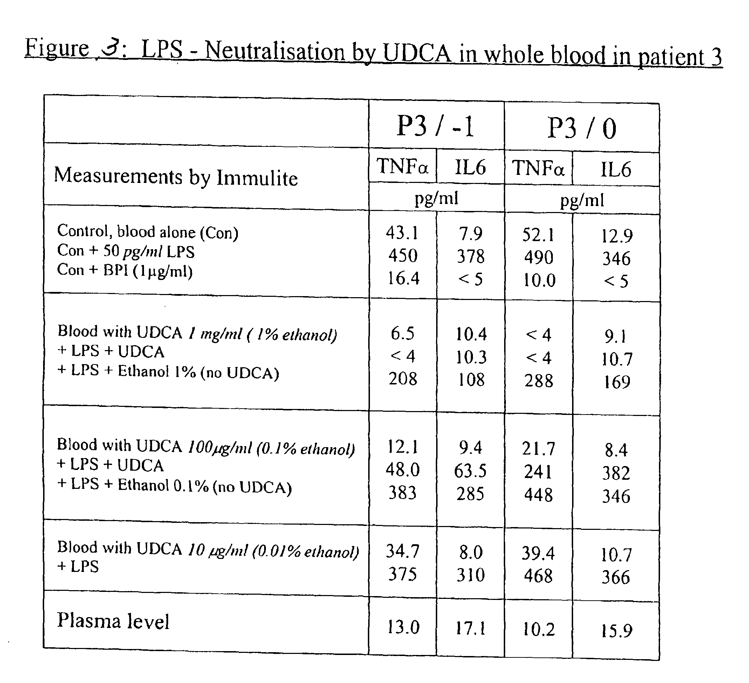

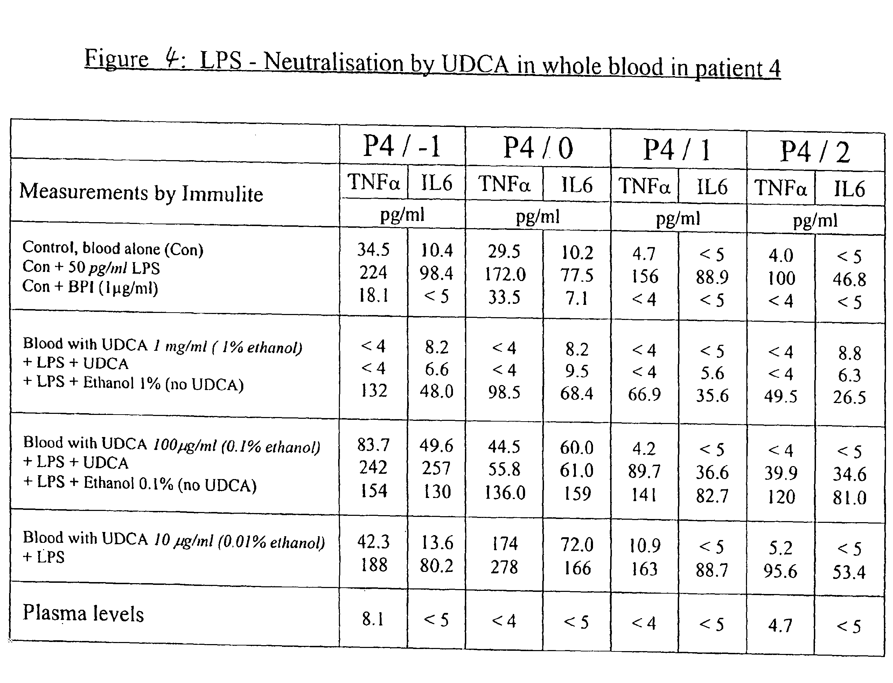

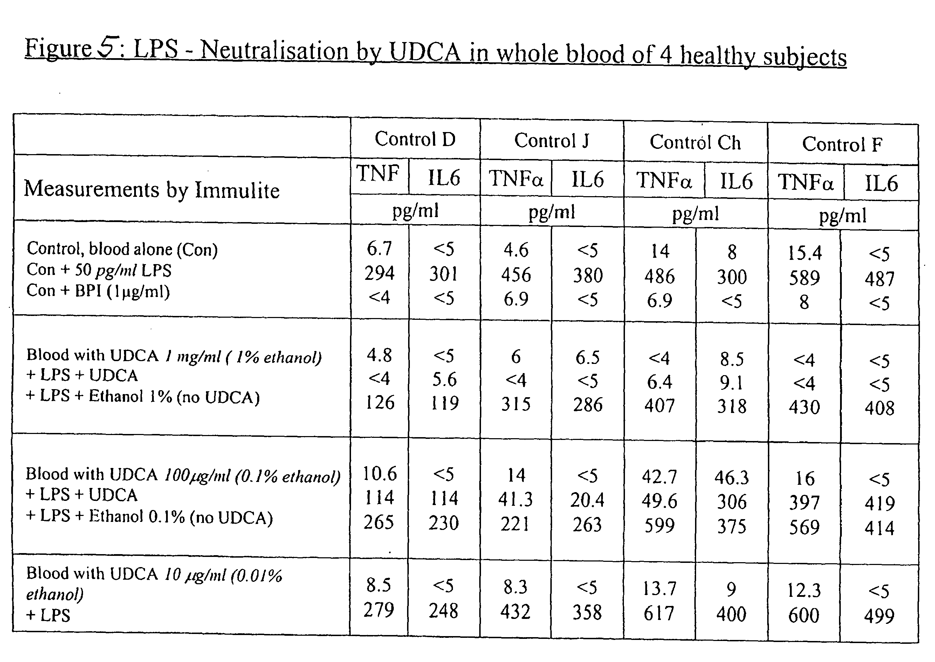

We have tested the ability of ursodeoxycholic acid (UDCA, FALK Pharma GmbH) and BPi to inhibit LPS-mediated TNF production in whole blood of patients with cachexia.
We studied 4 patients with cachexia due to liver cirrhosis. The patients had all weight loss >7.5% compared to their previous normal weight. In 3 of the 4 patients had a alcoholic aetiology. All patients were studied twice on 2 subsequent days (day "-1" and day "0"), see Figure 1 to 4.
**Methods:** Heparinized whole blood was diluted 1:10 with medium +/- LPS (50 pg/ml), +/- BPI (1 µg/ml), and +/- UDCA (1 µg/ml - 1 mg/ml) according to the manufactorer's recommendation (Milenia whole blood assay ; DPC Biermann, Bad Nauheim, Germany) and incubated for 4 hours at 37°C. In the supernatant, we assessed concentrations of TNF and IL-6 using the semiautomated Immulite system (DPC-Biermann, Bad Nauheim, Germany).
**Results**: In patients with cachexia due to liver cirrhosis spontaneous ("Control" data) and LPS-stimulated production of TNF and IL6 is significantly elevated compared to that of healthy subjects, see Figure 5. LPS-stimulated cytokine production was inhibited by UDCA independently of the effects of the ethanol solution. The detailed results are presented in Figure 1 to 4. 1 mg/ml UDCA reduced LPS-stimulated TNF production on average by >99% and IL6 production by 97% (ethanol 1% alone on average only by 38% for TNF and 43% for IL6). 100 µg/ml UDCA reduced LPS-stimulated TNF and IL6 production by 42% and 13%, respectively, ethanol 0.1% alone on average only 9% for TNF and IL6 production increased by 18% for ethanol alone).

BPi (1 µg/ml) reduced significantly the spontaneous production of TNF and IL6 of whole blood of patients with cachexia due to liver cirrhosis. In 8 experiments 6 times TNF and IL6 levels, respectively, were lowered by at least 5 pg/mi or towards non-detectability, and only in 2 cases TNF and IL6 levels remained stable (p<0.05 for changes).
**Conclusion:** This is the first documentation that LPS-stimulated cytokine production of whole blood of patients with cachexia can be inhibited by in vitro application of ursodeoxycholic acid (UDCA). This is the first documentation that spontaneous production of inflammatory cytokines in whole blood of patients with cachexia can be inhibited by application of BPi in vitro.

### Example 2:

We have tested the ability of the therapeutic application of ursodeoxycholic acid (UDCA, FALK Pharma GmbH) to lower plasma levels of TNF and IL6 and to lower spontaneous and LPS-stimulated whole blood cytokine production in patients with cachexia.
We studied in 2 patients with cachexia due to liver cirrhosis plasma cytokine levels after treatment with 3 times 250 mg daily UDCA (FALK Pharma GmbH). The patients had weight loss >7.5% compared to their previous normal weight. The patients were studied at baseline prior to the treatment on 2 subsequent days (day "-1" and day "0"), and then they were restudied on day 1 ("1"), day 2 ("2"), and day 5 ("5"), see Figure 9 and 12.
**Methods:** Heparinized whole blood was diluted 1:10 with medium +/- LPS (50 pg/ml), +/- BPI (1 µg/ml), and +/- UDCA (1 µg/ml - 1 mg/ml) according to the manufactorer's recommendation (Milenia whole blood assay ; DPC Biermann, Bad Nauheim, Germany) and incubated for 4 hours at 37°C. In the supernatant and in plasma, we assessed concentrations of TNF and IL-6 using the semiautomated Immulite system (DPC-Biermann, Bad Nauheim, Germany).
**Results:** Only patient 1 showed elevated plasma levels at baseline (Figure 9). During 5 days of treatment plasma levels of TNF were lower. In patient 4 we were able to reassess whole blood TNF and IL6 production after 1 and 2 days of treatment with UDCA. Spontaneous production of TNF and IL6 in whole blood was reduced substantially to almost undetectable levels. After 2 days of UDCA treatment LPS-stimulated cytokine production was found to be lowered by 43.5% for TNF and by 39.6% for IL6.
**Conclusion:** This is the first documentation that LPS-stimulated cytokine production of whole blood of patients with cachexia can be inhibited by in vivo therapeutic application of ursodeoxycholic acid (UDCA). This is the first documentation that plasma levels of TNF alpha of patients with cachexia can be inhibited by application of BPi.

### Example 3: Endotoxin in cachectic patients with liver cirrhosis.

It has never been studied, whether endotoxin (LPS) or a marker of endotoxaemia may be raised in patients with liver cirrhosis who suffer from cachexia. Plasma levels of soluble CD14 (sCD14) can reflect the history of LPS - cell interaction (Anker et al,., Am J Cardiol 1997; ;79:1426-1430.).
We investigated in 46 patients with liver cirrhosis (54±12 years, female 15, male 31, Child A:B:C=24:13:9), alcoholic aetiology in 32 patients) resting energy expenditure (REE, indirect calorimetry), food intake diaries, fat mass (skin fold thickness and calculation according to standard formulae) and body cell mass (BCM, body impedance, Data Input 2000, USA). Soluble CD14 was measured by ELISA (R&D Systems). The majority of patients had a BCM of<33% of body weight (mean±standard deviation: 25±7%, median 33%, range 11.8 - 41.9%). Plasma sCD14 levels were significantly increased in patients (mean±standard deviation: 4045±623 pg/ml, median 3920 pg/ml, range 2960 - 5460 pg/ml) compared to sCD14 levels of healthy individuals (mean: 2714 pg/ml, upper limit of normal 3711 pg/ml, as published in Anker et al,., Am J Cardiol 1997; ;79:1426-1430).
The patients with low BCM relative to their body weight must be considered to suffer from wasting disease, which was the majority in this study (63%of patients had a BCM <35%/kg body weight). The majority of patients in this study were metabolically catabolic as evidenced by a REE/BCM coefficient of 67±19 kcal/kg BCM (range 43-163, normal range in healthy subjects: 45 - 55 kcal/kg).
The strongest correlation that we found was between the degree of wasting (BCM per kg body weight) and the marker of endotoxaemia, i.e. soluble CD14 (r=-0.565, p<0.001). This means, the lower the relative BCM (i.e. the more cachectic) a patient was the higher the were also the sCD 14 plasma levels. Plasma levels of sCD 14 also correlated closely and directly with the degree of catabolic energetic/metabolic status (i.e. the REE/ BCM coefficient), r=0.549, p<0.001.
**Conclusion:** This is the first study suggesting that endotoxin (LPS) levels in patients with liver cirrhosis may be particularly high in patients with cachexia. This study also suggests that endotoxin (LPS) is causally related to the characteristics of the cachexia syndrome in liver cirrhosis, i.e. reductions in muscle tissue and increases in metabolic rate.

### Example 4: LBP in cachectic patients due to liver cirrhosis.

We have studied LBP plasma levels in 6 patients with cachexia due to liver cirrhosis. The patients had weight loss >7.5% compared to their previous normal weight. The disease aetiology was thought to be alcoholic in 4 cases and non-alcoholic in 2 cases. In non of these patients increased LBP levels were found (all below 20 µg/ml). High levels LBP can (together with lipoproteins) block LPS mediated production of inflammatory cytokines. We conclude that LBP is lacking in patients with cachexia due to liver cirrhosis, and that the application of LBP, possibly together with lipoproteins, could counteract the inflammatory status seen in these patients.

## Claims

1. Use of a compound for the manufacture of a medicament for treatment prevention or amelioration of endotoxin-mediated immune activation in body wasting or cachexia or in a patient with liver cirrhosis, chronic obstructive pulmonary disease, chronic renal failure, diabetes, rheumatoid arthritis wherein the compound is able to reduce the production, absorption and/or the effect of an endotoxin (lipopolysaccharide; LPS) preferably through binding to an endotoxin (lipopolysaccharide; LPS) molecule and/or reducing the available endotoxin in the patient, wherein the compound is
a) a bile acid; or
b) a bile acid wherein the bile acid is any one of ursodesoxycholic acid, chemodeoxycholic acid, dehydrocholic acid, cholic acid and deoxycholic acid; or
c) a LPS binding protein; or
d) a bactericidal/permeability increasing protein (BPI); or
e) a lipoprotein, for instance, low densitiy lipoprotein (LDL), high density lipoprotein (HDL), very low density lipoprotein (VLDL), apolipoprotein (a), a lipoprotein mixture; or
f) an antibody capable of binding to endotoxin (lipopolysaccharide; LPS); or
g) an antibody able to bind to the CD 14 receptor; or
h) a soluble CD14 receptor.

2. Use of a compound according to claim 1 wherein the compound is administered orally.

3. Use of a compound according to claim 1 wherein the compound is administered intravenously.

4. Use of a compound according to claim 1 wherein the compound is administered rectally.

## Patentansprüche

1. Verwendung eines Stoffs zur Herstellung eines Arzneimittels für die Behandlung, Vorbeugung oder Besserung von Endotoxin-vermittelter Immunaktivierung bei körperlicher Auszehrung oder Kachexie eines Patienten mit Leberzirrhose, chronisch obstruktiver Lungenerkrankung, chronischer Nierenfehlfunktion, Diabetes, rheumatoider Arthritis, wobei der Stoff geeignet ist, die Produktion, Absorption und/oder den Effekt eines Endotoxins (Lipopolysaccharid; LPS) zu reduzieren vorzugsweise durch Bindung an ein Endotoxin (Lipopolysaccharid; LPS) Molekül und/oder Reduzierung des vorhandenen Endotoxins in dem Patienten, wobei der Stoff ist
a) eine Gallensäure; oder
b) eine Gallensäure, wobei die Gallensäure eine beliebige ist von Ursodesoxycholsäure, Chemodeoxycholsäure, Dehydrocholsäure, Cholsäure und Deoxycholsäure; oder
c) ein LPS bindendes Protein; oder
d) ein bakterizid/Permeabiltäts-steigerndes Protein (BPI); oder
e) ein Lipoprotein, bspw. niedrige Dichte Lipoprotein (LDL), hohe Dichte Lipoprotein (HDL), sehr niedrige Dichte Lipoprotein (VLDL), Apolipoprotein (a), ein Lipoproteingemisch; oder
f) ein Antikörper, imstande an Endotoxin (Lipopolysaccharid; LPS) zu binden; oder
g) ein Antikörper, imstande an den CD14 Rezeptor zu binden; oder
h) ein löslicher CD14 Rezeptor.

2. Verwendung eines Stoffs gemäß Anspruch 1, wobei der Stoff oral verabreicht wird.

3. Verwendung eines Stoffs gemäß Anspruch 1, wobei der Stoff intravenös verabreicht wird.

4. Verwendung eines Stoffs gemäß Anspruch 1, wobei der Stoff rektal verabreicht wird.

## Revendications

1. Utilisation d'un composé pour la fabrication d'un médicament pour le traitement, la prévention ou l'amélioration de l'activation immune, induite par endotoxine, dans l'amaigrissement du corps ou la cachexie chez un patient, atteint de cirrhose du foie, de maladie pulmonaire obstructive chronique, de défaillance rénale chronique, de diabète, d'arthrite rhumatoïde, dans laquelle le composé est apte à diminuer la production, l'absorption et / ou l'effet d'une endotoxine (lipopolysaccharide ; LPS), de préférence par liaison avec une molécule d'endotoxine (lipopolysaccharide ; LPS) et / ou une diminution de l'endotoxine, contenue dans le corps du patient, dans laquelle le composé est
a) un acide biliaire ou
b) un acide biliaire, dans lequel l'acide biliaire est un acide quelconque parmi l'acide ursodésoxycholique, l'acide chémodéoxycholique, l'acide déhydrocholique, l'acide cholique et l'acide déoxycholique ou
c) une protéine de liaison du LPS ou
d) une protéine bactéricide / augmentant la perméabilité (BPI) ou
e) une lipoprotéine, par exemple : une lipoprotéine de basse densité (LDL), une lipoprotéine de haute densité (HDL), une lipoprotéine de très basse densité (VLDL), une apolipoprotéine (a), un mélange de lipoprotéines ou
f) un anticorps, capable de se lier à une endotoxine (lipopolysaccharide ; LPS) ou
g) un anticorps, apte à se lier avec le récepteur CD14 ou
h) un récepteur CD14 soluble.

2. Utilisation d'un composé selon la revendication 1, dans laquelle le composé est administré par voie orale.

3. Utilisation d'un composé selon la revendication 1, dans laquelle le composé est administré par voie intraveineuse.

4. Utilisation d'un composé selon la revendication 1, dans laquelle le composé est administré par voie rectale.
